(19) Europäisches Patentamt
European Patent Office
Office européen des brevets

(11) Veröffentlichungsnummer: **0 056 981**
**A1**

# (12) EUROPÄISCHE PATENTANMELDUNG

(21) Anmeldenummer: 82100383.7

(22) Anmeldetag: 21.01.82

(51) Int. Cl.³: C 07 C 69/63
C 07 C 67/30, B 01 J 31/02

(30) Priorität: 27.01.81 DE 3102516

(43) Veröffentlichungstag der Anmeldung: 04.08.82 Patentblatt 82/31

(84) Benannte Vertragsstaaten: AT BE CH DE FR GB IT LI NL SE

(71) Anmelder: HOECHST AKTIENGESELLSCHAFT
Postfach 80 03 20
D-6230 Frankfurt/Main 80(DE)

(72) Erfinder: Koch, Manfred, Dr.
Kreuzheck 2
D-6239 Eppstein/Taunus(DE)

## (54) Verfahren zur Herstellung von optisch aktiven 2-Chlorpropionsäureestern.

(57) Verfahren zur Herstellung von optisch aktiven 2-Chlorpropionsäureestern durch Zersetzung von optisch aktiven 2-Chlorsulfinoxypropionsäureestern in Gegenwart von quaternären Ammonium- bzw. Phosphoniumsalzen als Katalysator.

EP 0 056 981 A1

Croydon Printing Company Ltd

Verfahren zur Herstellung von optisch aktiven 2-Chlorpropionsäureestern

Die vorliegende Erfindung betrifft ein Verfahren zur Herstellung von optisch aktiven 2-Chlorpropionsäureestern durch Zersetzung von optisch aktiven 2-Chlorsulfinoxypropionsäureestern in Gegenwart von Katalysatoren.

Es ist bekannt, Alkohole und Hydroxy-carbonsäuren durch Umsetzung mit molaren Mengen von Pyridin und Thionylchlorid in entsprechende Chlorverbindungen zu überführen (Darzens, C.r. 152 (1911), S. 1314 u. 1601).

Die Reaktion verläuft nach folgendem Schema:

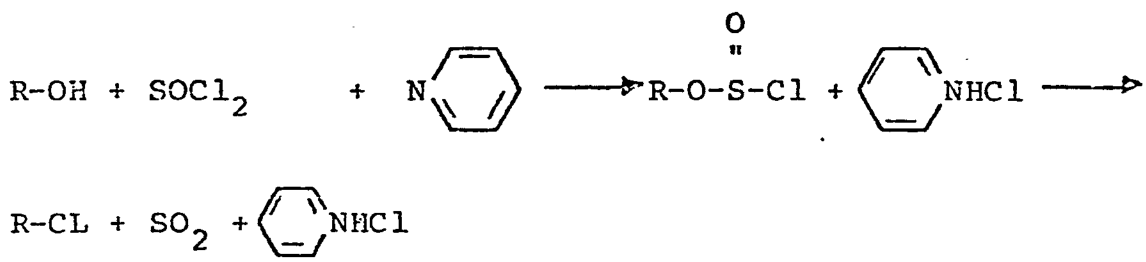

Nach diesem Verfahren erhielten Niemann et al. (J. org. Chem. 8 (1943), S. 401), ausgehend von racemischem Milchsäuremethylester 2-Chlor-propionsäuremethylester in einer Ausbeute von 71 % d. Th., ebenso Gerrard et al. (J. Chem. Soc. 1937 I, S. 153) ausgehend von D-Milchsäureäthylester in 70 %-iger Ausbeute L-2-Chlorpropionsäureäthylester mit einer spezifischen Drehung

$[\alpha]_D^{17} = -18,1°$ ($\alpha_{589}^{17°C} = -19,56°$)

entsprechend einer optischen Reinheit von ca. 90%.

Wegen der schlechten Ausbeute, aber auch wegen der ungenügenden optischen Reinheit der erhaltenen 2-Chlorpropionsäureester ist dieses Verfahren nicht für eine großtechnische Herstellung optisch hochreiner 2-Chlorpropionsäureester geeignet. Nachteilig ist ferner der hohe Anteil an Base im Reaktionsprodukt, die auf umständliche Weise zurückgewonnen werden muß.

Es ist weiter bekannt, daß die Zersetzung des in obiger Gleichung intermediär gebildeten Chlorsulfinsäureesters auch mit katalytischen Mengen Base (0.1 Mol pro Mol Hydroxykomponente bzw. Thionylchlorid) bewerkstelligt werden kann. (Houben-Weyl, Band VI/2 S. 435).

So erhielten Liebermann (Nature 160 (1947), S. 903) und Gerrard (Nature 159 (1947) S. 263) bei der Umsetzung von racemischem Milchsäureäthylester und Thionylchlorid unter Zusatz einer katalytischen Menge Pyridin 2-Chlorpropionsäureäthylester in einer Ausbeute von annähernd 95 % d. Th. Bei dieser Verfahrensweise kann der entstandene 2-Chlorpropionsäureester direkt aus dem Reaktionsgemisch abdestilliert werden. Damit bietet dieses Verfahren aufgrund der hohen Ausbeute, der technisch einfachen Durchführung und der Umweltfreundlichkeit eine Reihe von großen Vorteilen gegenüber der Umsetzung mit molaren Mengen Base.

Bei der Übertragung dieses nur für racemischen 2-Chlorpropionsäureäthylester beschriebenen Verfahrens auf optisch aktive 2-Chlorpropionsäureester ausgehend von optisch aktiven D- oder L-Milchsäureestern wurde jedoch festgestellt, daß zwar die gute chemische Ausbeute erhalten bleibt, aber die optische Aktivität größtenteils verloren geht. Der durch Umsetzung von L-Milchsäuremethylester, Thionylchlorid und katalytischen Mengen Pyridin nach dem Verfahren von Liebermann erhaltene 2-Chlorpropionsäuremethylester wies eine optische Reinheit von nur ca. 55 % d. Th. auf; der Verlust an optischer Reinheit betrug also mehr als 40 % d. Th. Damit ist diese Verfahrensweise zur Herstellung optisch aktiver 2-Chlorpropionsäureester ebenfalls nicht geeignet.

Es wurde nun überraschend festgestellt, daß es durch Verwendung von Phasentransfer-Katalysatoren gelingt, optisch aktive D- und L-Milchsäureester in hoher chemischer Ausbeute (92 - 96 % d. Th.) und hoher optischer Reinheit (95 - 98 % d. Th.) in entsprechende L- bzw. D-2-Chlorpropionsäureester zu überführen.

Gegenstand der Erfindung ist somit ein Verfahren zur Herstellung von optisch aktiven 2-Chlorpropionsäureestern der allgemeinen Formel I,

ClCH-COOR<br>
 |  (I)<br>
$CH_3$

worin R einen ($C_1$-$C_6$)-Alkylrest bedeutet, durch Zersetzung von optisch aktiven 2-Chlorsulfinoxypropionsäureestern der Formel II ,

O  $CH_3$<br>
‖  |<br>
ClS-O-CH-COOR  (II)

worin R die Bedeutung wie in Formel I hat, in Gegenwart von Katalysatoren, dadurch gekennzeichnet, daß man als Katalysatoren Oniumsalze (quaternäre Ammonium- bzw. Phosphoniumsalze) verwendet.

Die nach dem erfindungsgemäßen Verfahren hergestellten optisch aktiven 2-Chlorpropionsäureester I sind wertvolle Zwischenprodukte zur Herstellung von optisch aktiven Wuchsstoffherbiziden oder herbiziden Phenoxyphenoxypropionsäurederivaten.

Bei dem erfindungsgemäßen Verfahren werden D- oder L-Chlorsulfinoxypropionsäureester der allgemeinen Formel II kontinuierlich in ein mit einem Zersetzungskatalysator versehenes, beheiztes und evakuiertes Reaktorgefäß eindosiert, wobei gleichzeitig der L- oder D-2-Chlorpropionsäureester zusammen mit Schwefeldioxyd abdestilliert. Der optisch aktive 2-Chlorpropionsäureester wird in einer gekühlten Vorlage aufgefangen und Schwefeldioxyd in einer nachgeschalteten Vorlage kondensiert bzw. in wässriger Lauge absorbiert.

Das Reaktorgefäß wird auf 60 - 140°C, vorzugsweise 80 - 120 °C beheizt, das angelegte Vakuum beträgt zwischen 10 und 150 Torr, vorzugsweise zwischen 20 und 80 Torr. Temperatur und Vakuum werden so einreguliert, daß der gebildete D- oder L-2-Chlorpropionsäureester sofort aus dem Reaktionsgefäß abdestilliert. Als Zersetzungskatalysatoren werden quaternäre Ammonium- und Phosphoniumsalze ("Oniumsalze"), bevorzugt Chloride verwendet.

Als Beispiele seien genannt: Benzyltributylammoniumchlorid, Benzyltriäthylammoniumchlorid, Tetraäthylammoniumchlorid, Tetrabutylammoniumchlorid, Di-($C_{16}$-$C_{18}$)-alkyl-dimethylammoniumchlorid, Tri-($C_{16}$-$C_{18}$)-alkyl-methylammoniumchlorid, Methyltri-($C_8$-$C_{10}$)-alkylammoniumchlorid, Trimethyl-phenylammoniumchlorid, Dimethylpiperidiniumchlorid, N-Benzyl-N-methylephedriniumchlorid, N-Dodecyl-N-methylephedriniumchlorid, Tetrabutylphosphoniumchlorid, Tetraoctylphosphoniumchlorid, Hexadecyltributylphosphoniumchlorid, N-Dodecylthiazoliumchlorid, N-Methylpyridiniumchlorid, N-Dodecylpyridiniumchlorid, Trioctylpropylammoniumchlorid.

Die erforderliche Menge des eingesetzten Katalysators ist äußerst gering. Sie beträgt zwischen $1 \cdot 10^{-4}$ bis $5 \cdot 10^{-3}$ Mol pro Mol Chlorsulfinoxypropionsäureester.

Der zur Umsetzung eingesetzte Chlorsulfinoxypropionsäureester der Formel II kann nach bekannten Methoden, z.B. durch Umsetzung von D- oder L-Milchsäureestern mit Thionylchlorid erhalten werden (Houben/Weyl Bd. VI/2, S. 431) und ungereinigt in die Zersetzungsreaktion eingesetzt werden.

Wird bei der Herstellung von II ein Überschuß an Thionylchlorid verwendet, so destilliert dieses mit dem 2-Chlorpropionsäureester aus dem Reaktorgefäß ab.

0056981

Der in der Vorlage kondensierte optisch aktive 2-Chlorpropionsäureester enthält kleine Mengen an gelöstem Schwefeldioxyd und gegebenenfalls geringe Mengen an Thionylchlorid. Er kann durch eine rektifizierende Destillation gereinigt werden.

Die Ausbeute an optisch aktivem 2-Chlorpropionsäureester beträgt 95 - 98 % d. Th. bezogen auf eingesetzten optisch aktiven 2-Chlorsulfinoxypropionsäureester bzw. 92 - 96 % d. Th. bezogen auf den eingesetzten optisch aktiven Milchsäureester. Die optische Reinheit des erhaltenen 2-Chlorpropionsäureesters liegt bei Verwendung von optisch reinem Milchsäureester bei 95 - 98 % d.Th.

Die Umsetzung verläuft unter nahezu quantitativer Konfigurationsumkehr. So wird aus einem D-2-Sulfinoxypropionsäureester ein L-2-Chlorpropionsäureester und aus einem L-2-Sulfinoxypropionsäureester ein D-2-Chlorpropionsäureester erhalten.

## Verfahrensbeispiele

### 1. Herstellung der 2-Chlorsulfinoxypropionsäureester (Ausgangsmaterial) (allgemeine Vorschrift)

1.0 Mol L- oder D-Milchsäureester werden zu 1.05 Mol Thionylchlorid bei Raumtemperatur unter Rühren zugetropft. Nach Beendigung der Chlorwasserstoffentwicklung beläßt man die klare Lösung 24 Stunden bei Raumtemperatur. Der so erhaltene rohe L- oder D-2-Chlorsulfinoxypropionsäureester wird ohne weitere Reinigung für das erfindungsgemäße Verfahren verwendet.

Eine Reinigung kann durch Destillation erfolgen. Im Fall von L-Milchsäuremethylester erhält man nach Destillation 181 g = 97 % d. Th. L-2-Chlorsulfinoxypropionsäuremethylester vom Siedepunkt 45°C/0.013 mbar und dem Berechnungsindex $n_D^{21.5}$ = 1.4646.

### 2. Beispiele zum erfindungsgemäßen Verfahren

#### Allgemeines

Die zur Zersetzung des 2-Chlorsulfinoxypropionsäureesters verwendete Apparatur besteht aus einem 100 ml Zweihalskolben, mit einem Tropftrichter (zur Dosierung des 2-Chlorsulfinoxypropionsäureesters) und einem über eine kurze Vigreuxkolonne verbundenen, mit einer Eis-Kochsalz-Mischung gekühlten Liebigkühler, an den zwei mit Eis-Kochsalz gekühlte Vorlagen (zum Auffangen des gebildeten 2-Chlorpropionsäureesters) und eine weitere auf -78°C gekühlte Vorlage zur Kondensierung von Schwefeldioxyd angeschlossen sind.

Die Apparatur wird mit Hilfe einer Wasserstahlpumpe evakuiert. Das Reaktorgefäß wird mit Hilfe eines Ölbades beheizt.

## 2.1. L-2-Chlorpropionsäuremethylester

474 g roher D-2-Chlorsulfinoxypropionsäuremethylester (herstellt durch Umsetzung von 2,4 Mol D-Milchsäuremethylester ($[\alpha]_D^{23^\circ}$ = 8.03 °) und 2.56 Mol Thionylchlorid nach Beispiel 1) werden zum auf 100°C beheizten und mit 0.75 g Tri-($C_6$-$C_8$-alkyl)-methyl-ammoniumchlorid (Präpagen WK) versehenen Reaktorgefäß innerhalb von 2 Stunden zudosiert. Die Dosierung erfolgt so, daß ein Vakuum von etwa 40 - 60 Torr aufrechterhalten wird und der entstehende L-2-Chlorpropionsäuremethylester (Kopftemperatur etwa 50°C, 50 mm) abdestilliert. Nach Beendigung der Zugabe wird der Inhalt der Vorlagen 1 und 2 vereinigt. Man erhält 306 g rohen L-2-Chlorpropionsäuremethylester. Im Reaktorgefäß verbleiben 1.7 g. Bei der Rektifikation des rohen L-2-Chlorpropionsäuremethylester zur Abtrennung von Schwefeldioxyd und geringen Mengen Thionylchlorid erhält man 279g ≙ 95 % d.Th. (bezogen auf D-Milchsäuremethylester) reinen L-2-Chlorpropionsäuremethylester mit dem Siedepunkt 42° / 36 mbar und einer spezifischen Drehung $[\alpha]_D^{20^\circ C}$ = -25,6° ( 1, 1.0).

## 2.2. L-2-Chlorpropionsäuremethylester

In analoger Weise wie in 2.1., jedoch mit 0.3 g Methyl-tri-($C_6$-$C_8$-alkyl)-ammoniumchlorid wurden erhalten: 276 g ≙ 93.9 % d.Th. L-2-Chlorpropionsäuremethylester mit der spezifischen Drehung $[\alpha]_D^{20^\circ}$ = -25.4° (1, 1.0)

2.3. L-2-Chlorpropionsäuremethylester

In analoger Weise wie in 2.1., jedoch mit 0.5 g Triäthylbenzylammoniumchlorid wurden erhalten: 280.6 g = 95.5 % d.Th. L-2-Chlorpropionsäuremethylester mit der spezifischen Drehung $[\alpha]_D^{22^\circ} = -25.7^\circ$ (1, 1.0)

2.4. L-2-Chlorpropionsäuremethylester

In analoger Weise wie in 2.1., jedoch mit 0.5 g Tetraoctylphosphoniumchlorid wurden erhalten: 278 g = 94.7 % d.Th. L-2-Chlorpropionsäuremethylester mit der spezifischen Drehung $[\alpha]_D^{22^\circ} = -25.3^\circ$ (1, 1.0)

2.5. D-2-Chlorpropionsäuremethylester

In analoger Weise, jedoch ausgehend von L-Milchsäuremethylester ($[\alpha]_D^{25^\circ} = -8.56^\circ$) und unter Verwendung von 0.5 g Tri-($C_8$-$C_{10}$-alkyl)-methylammoniumchlorid wurden erhalten: 276.3 g = 94 % d.Th. D-2-Chlorpropionsäuremethylester mit der spezifischen Drehung $[\alpha]_D^{22^\circ} = +26.3^\circ$ (1, 1.0)

2.6. D-2-Chlorpropionsäureäthylester

In analoger Weise, jedoch ausgehend von käuflichem, redestillierten L-Milchsäureäthylester $[\alpha]_D^{24^\circ} = -10.6^\circ$ (1, 1.0) und unter Verwendung von 0.75 g Tri-($C_6$-$C_8$-alkyl)-methylammoniumchlorid wurden erhalten: 306 g = 93.3 % d.Th. D-2-Chlorpropionsäureäthylester mit der spezifischen Drehung $[\alpha]_D^{20^\circ} = +19.1^\circ$.

Vergleichsversuche

1. D-2-Chlorpropionsäuremethylester
[nach Liebermann Nature Vol. 160 (1947) S. 903]

0.5 Mol Thionylchlorid (59.5 g) werden unter Durchleiten eines leichten $N_2$-Storms zu 1.0 Mol L-Milchsäuremethylester [104 g ($[\alpha]_D^{21.5^\circ}$ = -8.44° (1, 1.0)] bei Raumtemperatur (externe Eiskühlung) zugetropft. Nach Beendigung der Chlorwasserstoffentwicklung werden weitere 0.6 Mol Thionylchlorid (71.5 g) zugesetzt und die Lösung 24 Stunden bei Raumtemperatur belassen. Nach Zugabe von 1 ml Pyridin wird 3 Stunden im Wasserbad bei etwa 95 - 100°C erhitzt, ausschließend D-2-Chlorpropionsäureester über eine Kolonne im Wasserstrahlvakuum abdestilliert. Man erhält 106 g = 86.5 % D-2-Chlorpropionsäuremethylester mit einer spezifischen Drehung von $[\alpha]_D^{22^\circ}$ = + 15.3° (1, 1.0)

2.1. L-2-Chlorpropionsäuremethylester
[nach Gerrard, Kenyon, Phillips (J. Chem. Soc. 1937 I, S. 153)]

0.398 Mol D-Milchsäuremethylester [41.4 g, $[\alpha]_D^{23^\circ}$ = 8.03° (1, 1.0)] und 0.405 Mol Pyridin (32 g)

werden in einem 250 ml Vierhalskolben vorgelegt und innerhalb 45 Minuten bei einer Innentemperatur von 20 - 30°C mit 0.405 Mol Thionylchlorid (48 g) versetzt. Beim Zutropfen von Thionylchlorid bildet sich ein dicker Brei von Pyridinhydrochlorid, so daß der Ansatz nach Zugabe von etwa 2/3 der Thionylchloridmenge nicht mehr rührbar ist. Nach beendeter Zugabe wird 1 Stunde bei 60°C erhitzt, abgekühlt und die Reaktionsmischung auf Eiswasser gegeben, anschließend mit insgesamt 400 ml Diäthyläther zweimal extrahiert. Diäthyläther wird über eine Kolonne bei Normaldruck abdestilliert, der zurückbleibende L-2-Chlorpropionsäuremethylester im Wasserstrahlvakuum, Siedepunkt 38°C, 21 Torr abdestilliert. Man erhält 35.6 g = 72.8 % L-2-Chlorpropionsäuremethylester mit der spezifischen Drehung $[\alpha]_D^{26^\circ C} = -23.5^\circ$ (1, 1.0)

Patentansprüche:

1. Verfahren zur Herstellung von optisch aktiven 2-Chlorpropionsäureestern der Formel I,

$$\begin{array}{l} \quad CH_3 \\ \quad \; | \\ ClCH\text{-}COOR \end{array} \qquad (I)$$

worin R einen $(C_1-C_6)$-Alkylrest bedeutet, durch Zersetzung von optisch aktiven 2-Chlorsulfinoxypropionsäureestern der Formel II,

$$\begin{array}{l} \quad O \quad\;\; CH_3 \\ \quad \| \qquad \; | \\ ClS\text{-}O\text{-}CH\text{-}COOR \end{array} \qquad (II)$$

worin R die Bedeutung wie in Formel I hat, in Gegenwart von Katalysatoren, dadurch gekennzeichnet, daß man als Katalysatoren Oniumsalze (quaternäre Ammonium- bzw. Phosphoniumsalze) verwendet.

2. Verfahren gemäß Anspruch 1, dadurch gekennzeichnet, daß man als Katalysatoren quaternäre Ammonium- bzw. Phosphoniumchloride verwendet.

3. Verfahren gemäß Anspruch 1 und 2, dadurch gekennzeichnet, daß man $1 \cdot 10^{-4}$ bis $5 \cdot 10^{-3}$ Mol Katalysator pro Mol 2-Chlorsulfinoxypropionsäureester verwendet.

4. Verfahren gemäß Anspruch 1 - 3, dadurch gekennzeichnet, daß man die Zersetzung bei Temperaturen von 60 - 120°C durchführt und gleichzeitig den gebildeten optisch aktiven 2-Chlorpropionsäureester unter Vakuum aus dem Reaktionsgemisch abdestilliert.

0056981

Nummer der Anmeldung

EP 82 10 0383.7

Europäisches Patentamt

# EUROPÄISCHER RECHERCHENBERICHT

| EINSCHLÄGIGE DOKUMENTE | | | KLASSIFIKATION DER ANMELDUNG (Int Cl.³) |
|---|---|---|---|
| Kategorie | Kennzeichnung des Dokuments mit Angabe, soweit erforderlich, der maßgeblichen Teile | betrifft Anspruch | |
| Y | GB - A - 2 051 808 (RHONE-POULENC AGROCHIMIE)<br>* Anspruch 1; Beispiel 1 *<br>& DE - A - 3 023 122<br>-- | 1,4 | C 07 C 69/63<br>C 07 C 67/30<br>B 01 J 31/02 |
| Y,P | GB - A - 2 055 802 (I.C.I.)<br>* Anspruch 1 *<br>---- | 1 | |

RECHERCHIERTE SACHGEBIETE (Int. Cl.³)

B 01 J 31/02
C 07 C 67/30
C 07 C 69/62
C 07 C 69/63

KATEGORIE DER GENANNTEN DOKUMENTE

X: von besonderer Bedeutung allein betrachtet
Y: von besonderer Bedeutung in Verbindung mit einer anderen Veröffentlichung derselben Kategorie
A: technologischer Hintergrund
O: nichtschriftliche Offenbarung
P: Zwischenliteratur
T: der Erfindung zugrunde liegende Theorien oder Grundsätze
E: älteres Patentdokument, das jedoch erst am oder nach dem Anmeldedatum veroffentlicht worden ist
D: in der Anmeldung angeführtes Dokument
L: aus andern Gründen angeführtes Dokument

&: Mitglied der gleichen Patentfamilie, übereinstimmendes Dokument

X Der vorliegende Recherchenbericht wurde für alle Patentansprüche erstellt.

| Recherchenort | Abschlußdatum der Recherche | Prufer |
|---|---|---|
| Berlin | 27-04-1982 | KNAACK |